# EUROPEAN PATENT APPLICATION

(11) **EP 4 194 824 A1**
(43) Date of publication of application: **14.06.2023**
(21) Application number: 22212329.1
(22) Date of filing: 08.12.2022
(51) Int. Cl.: G01J 5/02

(54) **PROBE COVER ARRAY AND SUPPORT**

(30) Priority: 09.12.2021 GB 202117803
(71) Applicant: Flexicare (Group) Limited, Mountain Ash, Mid Glamorgan CF45 4ER (GB)
(72) Inventor: MORRIS, Tomos, Mid Glamorgan, CF45 4ER (GB); BENNION, Nicholas, Mid Glamorgan, CF45 4ER (GB)
(74) Representative: Mewburn Ellis LLP

(57) **Abstract**

In various embodiments, the present invention provides probe cover arrays (such as tympanic probe cover arrays) support trays for such probe cover arrays, and kits comprising such probe cover arrays and support trays. The probe cover arrays comprise a plurality of probe covers connected to a frame via one or more frangible links. The support trays comprise a body and a plurality of wells formed in the body, each well being configured for receipt of a probe cover. Probe cover arrays, support trays and kits according to the present invention may have improved ease of use and/or improved packing efficiency in comparison to known products.

## Description

This application claims priority from GB 2117803.3 filed 9 December 2021, the contents and elements of which are herein incorporated by reference for all purposes.

### Field of the Invention

The present invention relates to a probe cover array, and a support for a probe cover array. It particularly, although not exclusively, relates to tympanic thermometer probe cover arrays - i.e. arrays of probe covers suitable for use in covering tympanic thermometer probes, and to support trays for such arrays.

### Background

The use of medical thermometers to measure a patient's body temperature is routine. Many different types of thermometer are available, however 'non-contact' thermometers are often considered to be particularly convenient for use in certain environments, due to the reduced potential for contamination risk.

One type of non-contact thermometer is a tympanic thermometer, or digital ear thermometer. Tympanic thermometers have a probe which is inserted into the ear and use IR to measure the temperature of the eardrum, and they are widely used in a variety of medical settings.

It is routine, especially in hospital/medical environments, that a disposable cover is used to cover the probe, the cover being discarded after each measurement to avoid contamination risks. Existing probes covers generally consist of a small plastic cone, shaped to fit closely over the thermometer's probe. They may have a thin translucent window closing off the narrow end of the probe where the IR passes.

To minimize handling contamination the covers can be provided side-by-side in preformed arrays or cassettes, in which multiple individual covers are connected detachably to one another and/or to a surrounding frame via thin frangible links. A cover can be put on the thermometer probe simply by pushing the probe into one of the covers (still in the array) and breaking the frangible links by pushing/turning slightly.

EP1857794B1 discloses one such probe cover cassette, the cassette comprising a frame and a plurality of tympanic thermometer probe covers arranged in at least one row, where each of the probe covers is releasably attached to the frame by at least one frangible connection. The cassette includes at least two longitudinal supports which extend longitudinally along the cassette, and a plurality of wall/arch-type cross braces connecting the longitudinal and positioned between adjacent releasable attached probe covers. The longitudinal and crossbrace supports are provided to resist deflection of the frame when the probe covers are detached from the frame by application of a detachment force to the probe covers of the array.

It is generally desirable to improve the packing efficiency of probe covers on a cassette, as more efficient use of space can e.g. reduce shipping and storage costs for the probe cover arrays. Furthermore, it is also desirable to provide probe cover arrays which are user-friendly in terms of their ease of loading a probe cover onto a tympanic probe.

The present invention has been devised in light of the above considerations.

### Summary of the Invention

Accordingly, in a first aspect, the present invention provides a probe cover array comprising a frame, and a plurality of probe covers connected to the frame via one or more frangible links, wherein the plurality of probe covers are arranged in at least two staggered rows, wherein the center-to-center spacing between each given probe cover and all probe covers directly adjacent said given probe cover is substantially equal.

The term "staggered rows" is used herein to define an arrangement in which the probe covers in one row are offset (staggered) with respect to the probe covers in an adjacent row. For example, the longitudinal axes of probe covers in a first row may be aligned with the midpoint between two directly adjacent probe covers in a second, directly adjacent, row. Note that the longitudinal axis of a given probe cover of the array of probe covers is generally perpendicular to the longitudinal axis of the probe cover array itself.

Where the center-to-center spacing between each given probe cover and all probe covers directly adjacent said given probe cover (including those probe covers directly adjacent, in an adjacent row of probe covers) is substantially equal, the probe covers can be said to be arranged in a hexagonal close packed arrangement.

In some cases, where the presence of the frangible links prevents direct touching of adjacent probe covers, the probe covers can be said to be arranged in a pseudo-hexagonal close packed arrangement - in other words, hexagonal close packed, other than for the spacing provided by the presence of the frangible links and/or other elements connecting frangible links. In these cases, the center-to-center spacing between each given probe cover and all probe covers directly adjacent said given probe cover may be at least 1, and no greater than 1.5 times the diameter of the given probe cover. In more preferred arrangements, the center-to-center spacing between each given probe cover and all probe covers directly adjacent said given probe cover is preferably no greater than 1.3 times the diameter of the given probe cover, no greater than 1.2 times the diameter of the given probe cover, more preferably no greater than 1.1 times the diameter of the given probe cover, or no greater than 1.05 times the diameter of the given probe cover. The diameter of the given probe cover may be measured at the widest point of the probe cover.

It has been found that for arrangements where the center-to-center spacing between each given probe cover and all probe covers directly adjacent said given probe cover is substantially equal, the packing efficiency of the probe cover array can be improved or maximized in comparison to arrangements where the center-to-center spacing between each given probe cover and all probe covers directly adjacent said given probe cover is not substantially equal. For example, in the arrangement of EP1857794B1, discussed above, a longitudinal intermediate support is provided between adjacent rows of probe covers, and so the centre-to-centre distance between adjacent probe covers in a first row and a second row is larger than the centre-to-centre distance between adjacent probe covers in a first row, because of the presence of the longitudinal intermediate support between the first and second rows. The arrangement of the present invention therefore achieves improved packing efficiency in comparison to this alternative known arrangement.

The number of rows of the probe cover array is not particularly limited. In some arrangements the probe cover array may comprise three or more rows of probe covers, four or more rows of probe covers, or five or more rows of probe covers. In some convenient arrangements, three rows of probe covers may be preferred.

The number of probe covers in each row of probe covers is not particularly limited. In some arrangements, each row of probe covers may comprise three or more probe covers, four or more probe covers, five or more probe covers, six or more probe covers, seven or more probe covers, eight or more probe covers, nine or more probe covers, or ten or more probe covers. The number of probe covers in each row of probe covers may be the same. Alternatively, in some arrangements, the number of probe covers in each row may vary between rows.

As set out above, each probe cover is connected to the frame via one or more frangible links. The term "attached to the frame via" does not necessarily mean that each given probe cover is directly connected to the frame via one or more frangible links. Rather, each probe cover may be either directly or indirectly connected to the frame via one or more frangible links. For example, for a probe cover that is indirectly attached to the frame via one or more frangible links, the probe cover may be attached to a first frangible link that attaches directly or indirectly (e.g. via another element) to one or more further probe covers, said further probe cover(s) being directly attached to the frame by further frangible link(s). In this way, there is an indirect connection from the probe cover to the frame via the first frangible link, further probe cover, and further frangible link.

The term "frangible link" is used herein to define an element or component of the probe cover array which is configured to break on application of a predetermined force. This force may sometime be referred to as a `detachment force'. In this way, as the probe covers are attached to the frame of the probe cover array via frangible links, the connection of the probe covers to the frame via the links can support the probe covers during storage, but the probe covers can readily be detached by a user by application of a detachment force (for example, when inserting a thermometer probe into a given probe cover for use).

The specific configuration and form of the frangible links is not particularly limited. However, in preferred arrangements, the frangible link may comprise a web of material. The dimensions of the frangible links may be relatively small in relation to the size of the probe covers. This can help to ensure that the detachment force required to break the frangible link(s) is suitably low. If the detachment force required to break the frangible link(s) is too high, the probe cover array may be bent or damaged during application of the detachment force.

In one preferred arrangement, the frangible links have at least 1 dimension that is 1 mm or less, for example, they may have at least 1 dimension that is 0.5mm or less. In some arrangements, the frangible links may be 1mm or less in two dimensions, or in three dimensions. In some preferred arrangements, the frangible links are approximately 0.5 mm wide, 1 mm long, and 1 mm thick.

The frangible links may comprise a mechanical stress raiser feature. This may reduce the force needed to break the frangible links. The mechanical stress raiser feature may comprise one or more notches or grooves provided on the frangible links.

The shape of the probe covers is not particularly limited. However, the probe covers may each comprise a tubular body portion, the body having a proximal end defining an opening configured for receipt of a distal end of a thermometer, and a distal end, distal to the proximal end.

The tubular body portion of the probe covers may be tapered. Preferably, the tubular body portion is tapered from the proximal end to the distal end. That is, the diameter of the tubular body portion may gradually reduce from the proximal end defining an opening configured for receipt of a distal end of a thermometer, and a distal end, distal to the proximal end. The tapered may be a continuous (i.e. smooth) taper. Alternatively, the taper may be a step-wise taper.

The diameter of the tubular body portion at the proximal end may be in a range of from 8 mm to 20 mm, preferably in a range of from 10 to 15 mm. For example, in some embodiments, the diameter of the tubular body portion at the proximal end may be about 9 mm, about 10 mm, about 11 mm, about 12 mm, about 13 mm, or about 14 mm. The diameter may be an internal diameter. In one preferred embodiment, the internal diameter of the tubular body portion at the proximal end is about 12 mm.

The diameter of the tubular body portion at the distal end may be in a range of from 1 mm to 10 mm, preferably in a range of from 2 to 7 mm. For example, in some embodiments, the diameter of the tubular body portion at the proximal end may be about 2 mm, about 3 mm, about 4 mm, about 5 mm, about 6 mm, or about 7 mm. The diameter may be an internal diameter. In one preferred embodiment, the internal diameter of the tubular body portion at the distal end is about 5.6 mm.

In preferred arrangements, the tubular body portion may be generally frusto-conical in shape. Providing a frusto-conical shape can allow the probe cover to be partially inserted into ear canals of different sized patients - for example, both children and adults. A frustro-conical shape may also allow the probe cover to closely fit a thermometer probe such as a tympanic thermometer probe. This can assist in retention of the probe cover on the thermometer probe during use.

In some arrangements, the probe cover may comprise one or more engagement features for engagement with corresponding engagement features provided on the thermometer probe. Accordingly, the probe cover may be releasably engagement with the thermometer probe via said engagement features. The engagement features may comprise clips or another suitable arrangement. The one or more engagement features may comprise one or more through-holes, depressions or raised features provided on the probe cover body. For example, one, two, three or four or more through-holes, depressions or raised features may be provided on the probe cover body. The size and/or shape of the engagement features may be selected to engage with one or more corresponding engagement features provided on the thermometer probe. The probe cover may be releasable from engagement with the thermometer probe via said engagement features by any suitable mechanism. For example, in one suitable arrangement, an engagement feature provided on the thermometer probe may be moveable in response to user input e.g. in response to a user pressing a button on the thermometer probe, said user input thereby resulting in disengagement of the probe cover and the thermometer probe.

Whilst provision of such engagement features on the probe cover may be advantageous, it is not essential, and in alternative arrangements, the probe cover may be retained on the thermometer probe by other means, e.g. by means of friction fit engagement alone.

In some arrangements, the distal end of the probe covers may comprise a membrane or window. The membrane or window, if present, may be substantially transparent to infrared radiation. For example, the membrane or window may comprise polyethylene or another IRtransparent polymer. Alternatively, the distal end may define a second opening in the tubular probe body.

The frame of the probe cover array may comprise peripheral sidewalls. The peripheral sidewalls may comprise two longitudinal wall portions which extend in a direction substantially parallel to the rows of probe covers. The peripheral sidewalls may comprise two lateral wall portions which extend in a direction substantially perpendicular to the rows of probe covers. The shape of the longitudinal wall portion(s) and the lateral wall portions(s) of the peripheral sidewalls of the frame is not particularly limited. In some arrangements, the peripheral sidewalls may comprise one or more curved wall portions connecting the longitudinal wall portion(s) and the lateral wall portion(s). The curved wall portions may follow the contours or general shape of an outer surface of the probe covers in the array. This may avoid the presence of sharp corners on the frame, and may furthermore provide a more aesthetically pleasing look for the probe cover array.

The probe cover array may comprise one or more support pins arranged between adjacent probe covers. The term "pin" is used herein to refer generally to an elongate member or element. The support pins may be arranged to provide a reaction force when a detachment force is applied to a probe cover of the plurality of probe covers - for example by engagement of the support pin with a supporting surface beneath the support pin. In this way, the support pins can act to provide a direct loading pathway to the surface on which the probe cover array is placed. This can reduce or eliminate the need to provide supporting/bracing members as part of the probe cover array, thereby minimizing the amount of material needed for the probe cover array. Accordingly, in such an arrangement, the probe cover array preferably does not comprise laterally- or longitudinally-extending bracing elements e.g. cross-bars or walls, located between the probe covers.

It is considered that provision of such support pins may provide technical effects extending beyond application in probe arrays where the center-to-center spacing between each given probe cover and all probe covers directly adjacent said given probe cover is substantially equal, and may be in fact be more generally applicable to other types of probe array.

Accordingly, in a second aspect, the present invention provides a probe cover array comprising a frame, and a plurality of probe covers connected to the frame via one or more frangible links, wherein the probe cover array comprises one or more support pins arranged between adjacent probe covers.

The support pin(s) may comprise elongate members or elements, wherein the longitudinal axis of the support pin(s) generally aligns with the longitudinal axis of a probe cover of the array of probe covers. In other words, the support pins may generally extend in the same direction as the probe covers in the array. Note that the longitudinal axis of a probe cover of the array of probe covers is generally perpendicular to the longitudinal axis of the probe cover array itself.

In some arrangements, the length of the support pins may be greater than or equal to the length of the tubular body portion of the probe covers. In other arrangements, the length of the support pins may be less than the length of the tubular body portion of the probe covers, and be configured to provide a reactionary force when the probe cover array is used in conjunction with a support tray (discussed in further detail below).

The specific shape of the support pins is not particularly limited. In some arrangements, the support pins may have a generally circular, square, rectangular, or triangular cross section. In some arrangements they may be prismatic in shape (i.e. having a constant cross section). In other arrangements, the cross-section of the support pins may vary along the length of the support pins. Where the cross-section of the support pins varies along the length of the support pins, it may vary continuously (e.g. the pins may be tapered), or may vary discontinuously (e.g. the pins may include a step-change in cross-sectional diameter.

A generally triangular cross-sectional shape may be preferred, as this may provide a suitably high second moment of area of the support pin in a lateral axis, thereby providing for increased bending stiffness of the support pins.

The probe covers in the probe cover array may be formed of the same material as the frangible links, and as the frame of the probe cover array. Alternatively, one or all of the probe covers, frangible links, and/or frame of the probe cover array may be formed from different materials. The components of the probe cover array may be formed from a polymeric material. This can provide for relatively low cost manufacture of the probe cover array. Suitable materials for components of the probe cover array include (but are not limited to): polypropylene, polyethylene, nylon, acrylonitrile butadiene styrene (ABS), and/or polycarbonate.

The probe cover array may be manufactured in any suitable manner. However, in one preferred arrangement, the probe cover array may be formed as a single, integrally moulded unit. The probe cover array may be formed by e.g. an injection moulding process.

As mentioned above, the probe cover array may be configured for use with a support tray. This applies to the probe cover arrays of both the first and second aspects.

Accordingly, in a third aspect, the present invention provides a support tray comprising a body and a plurality of wells formed in the body, each well being configured for receipt of a probe cover, wherein the support tray is configured to receive and support a probe cover array according to the first or second aspects of the present invention.

The support tray body may comprise peripheral sidewalls including two longitudinal wall portions. The peripheral sidewalls may further comprise two lateral wall portions which extend in a direction substantially perpendicular to the direction in which the longitudinal wall portions extend. Where the support tray comprises rows of wells, the longitudinal wall portions may extend in a direction substantially parallel to the rows of wells. The shape of the longitudinal wall portion(s) and the lateral wall portions(s) of the support tray body is not particularly limited. In some arrangements, the peripheral sidewalls may comprise one or more curved wall portions connecting the longitudinal wall portion(s) and the lateral wall portion(s). The curved wall portions may follow the contours or general shape of an outer surface of the wells in the support tray body. This may avoid the presence of sharp corners on the support tray, and may furthermore provide a more aesthetically pleasing look for the support tray body.

The support tray may be configured to provide a close-fit arrangement between the support tray and the probe cover array. In one arrangement, at least a part of the peripheral sidewalls of the support tray may be configured for engagement with at least a part of the frame of the probe cover array which the support tray is configured to receive and support. In this way, the probe cover array and the support tray can be readily assembled to provide a joined assembly having a relatively stable connection between the probe cover array and the support tray.

In one possible arrangement, the peripheral sidewalls of the support tray may comprise a recessed portion which extends around the periphery of the support tray. The recessed portion may be shaped to conform with at least part of the frame of the probe cover array. Preferably, the recessed portion is shaped to conform with a sidewall surface (e.g. an inner sidewall surface) of peripheral sidewalls of the probe cover array which the support tray is configured to receive and support.

The number of rows of wells in the support tray is not particularly limited. In some arrangements the support tray may comprise three or more rows of wells, four or more rows of wells, or five or more rows of wells. In some convenient arrangements, three rows of wells may be preferred.

The number of wells in each row of wells is not particularly limited. In some arrangements, each row of wells may comprise three or more wells, four or more wells, five or more wells, six or more wells, seven or more wells, eight or more wells, nine or more wells, or ten or more wells.

Preferably the number and arrangement of wells in the support tray substantially corresponds to the number and arrangement of probe covers in the probe cover array.

When the support tray is configured to support a probe cover array according to the first aspect of the invention, the plurality of wells may be arranged in at least two staggered rows, wherein the center-to-center spacing between each given well and all wells directly adjacent said given well is substantially equal.

The same considerations and optional features set out above in relation to the number, arrangement, and spacing of the probe covers in the probe cover array of the first aspect are equally applicably to the number, arrangement, and spacing of the wells in the support tray. In particular, the center-to-center spacing between each given well and wells directly adjacent said given well may be at least 1, and no greater than 1.3 times the diameter of the given well, no greater than 1.2 times the diameter of the given well, more preferably no greater than 1.1 times the diameter of the given well, or no greater than 1.05 times the diameter of the given well. The diameter of the given well may be measured at the widest point of the well.

When the support tray is configured to support a probe cover array according to the second aspect of the invention, the support tray may comprise one or more location points arranged between adjacent wells, for location of a support pin of the probe cover array.

One or more (or all) of the location points may comprise a depression, socket, or through-hole formed in the support tray body. This can assist in location of the support pins at the location points.

The location points may comprise a sloped lead-in surface surrounding the depression, socket, or through-hole. Provision of a sloped lead-in surface can assist in location of the support pin(s) in the location point(s) during alignment of the probe cover array and the support tray.

Where the location point(s) comprise a depression or socket, preferably the depth of the depression or socket is less than the length of the support pin(s). In this way, the support pins may engage the bottom of the depression or socket when they are located in the depression/socket. In such an arrangement, the bottom of the depression/socket therefore acts to provide a supporting surface beneath the support pin which can provide a reaction force when a detachment force is applied to a probe cover of the plurality of probe covers by engagement of the support pin with said supporting surface.

Where the location point(s) comprise a through-hole formed in the support tray body, the diameter of the through hole may be selected such that only a portion of a support pin can pass through the through hole. Such arrangements may require less complex molding than arrangements where the location point(s) comprise a depression or socket, whilst still providing the required location/support function.

Arrangements where one or more (or all) of the location point(s) comprise a through-hole formed in the support tray body may be particularly preferred for use with support pins where the cross-sectional shape of the support pins varies along the length of the support pins - e.g. wherein the support pins are tapered, or where they include a step-change in cross-sectional diameter. For example, where the support pins are tapered, the diameter of the through-hole may be selected to be equal to the diameter of the support pins at a given position along the length of the support pins, for example at 10%, 20%, 30%, 40%, 50%, 60%, 70%, 80% or 90% along the length of the support pin, from a distal (e.g. free) end of the support pin. Where the support pins comprise a step-change in cross-sectional diameter, the diameter of the through-holes may be selected to allow the narrower portion of the support pin to pass through the hole, but not allow the wider portion of the support pin to pass through the hole. In this case, the step surface between the narrower and wider portions of the support pin can act as an engagement surface for engaging with a surface of the support tray body adjacent the through-hole.

The shape of the location point(s) (e.g. the shape of the depression(s), socket(s) and/or through-holes, where applicable) may be selected to correspond with the shape of the support pins(s) of a probe cover array which the support try is configured to support.

The shape of the location point(s) and/or the support pin(s) may be selected such that it is only possible to locate a support pin in a corresponding location point in a predetermined number of selected orientations - i.e. it may be preferred that the location point and/or corresponding support pin do not have a circular cross section.

In some arrangements, the depression(s), socket(s) and/or through-holes constituting the location point(s) may be approximately square, rectangular, or triangular in shape. They may be configured for engagement with corresponding support pin(s) having approximately square, rectangular, or triangular cross-sections.

The shape of the wells of the support tray is not particularly limited other than that they must be configured for receipt of a probe cover. The wells may have a proximal end defining an opening configured for receipt of a probe cover, and a distal end, distal to the proximal end. The distal end may be open or closed.

In some arrangements, the wells may be tapered along their depth. They may be tapered from the proximal end to the distal end. That is, the diameter of the wells may gradually reduce from the proximal end defining an opening configured for receipt of a probe cover, and a distal end, distal to the proximal end. In preferred arrangements, the wells may be generally frusto-conical in shape.

The diameter of the well at the proximal end may be in a range of from 8 mm to 20 mm, preferably in a range of from 10 to 17 mm. For example, in some embodiments, the diameter of the tubular body portion at the proximal end may be about 11 mm, about 12 mm, about 13 mm, about 14 mm, about 15 mm, or about 16 mm. The diameter may be an internal diameter. In one preferred embodiment, the internal diameter of the tubular body portion at the proximal end is about 14.06 mm..

The diameter of the well at the distal end may be in a range of from 5 mm to 15 mm, preferably in a range of from 6 to 10 mm. For example, in some embodiments, the diameter of the tubular body portion at the proximal end may be about 7 mm, about 8 mm, or about 9 mm. The diameter may be an internal diameter. In one preferred embodiment, the internal diameter of the tubular body portion at the distal end is about 8.49 mm.

The material of the support tray is not particularly limited. However, in preferred arrangements, the support tray is formed from a polymeric material. This can provide for relatively low cost manufacture of support tray. Suitable materials for components of the support tray include (but are not limited to): polypropylene, polyethylene, nylon, acrylonitrile butadiene styrene (ABS), and/or polycarbonate. In some cases it may be convenient to form the support tray from the same material as the probe cover array.

The support tray may be manufactured in any suitable manner. However, in one preferred arrangement, the support tray is formed as a single, integrally moulded unit. The support tray may be formed by e.g. an injection moulding process.

In a fourth aspect, the present invention provides a kit of parts comprising a support tray according to the third aspect of the invention, and one or more probe cover arrays according to the first or second aspects of the present invention.

The support tray may have greater rigidity than the probe cover array(s). In this way, the support tray can readily hold the probe cover array steady and provide a stable reaction when a probe cover is detached from the probe cover array by application of a detachment force (e.g. when a user inserts a thermometer probe into a given probe cover for use).

In some arrangements, the probe cover array and/or the support tray may comprise one or more engagement feature(s) configured to retain the probe cover array on the support tray during use. The engagement feature(s) may be configured to retain the probe cover array on the support tray during detachment and removal of a probe cover from the probe cover array by a user. This can both assist in efficient detachment of a single probe cover from the probe cover array during use by a user, as well as ensuring continued alignment of the probe cover array with the support tray during use. For example, the presence of such engagement features may allow for an upwards detachment force to be applied to a probe cover in the array to break the frangible link(s) and thereby remove the probe cover from the array, without undesirably lifting the remainder of the probe cover array.

The engagement feature(s) may be provided in any suitable form. In some arrangements, the probe cover array and the support tray may comprise corresponding engagement features. The engagement features may engage by snap-fit connection. Alternatively, the engagement features may engage by providing an interference fit/friction fit between corresponding engagement features.

One of the probe cover array and the support tray may comprise a cantilever snap fit configured to engage with a corresponding engagement feature such as a recess or flange provided on the other of the probe cover array and support tray. The cantilever snap fit and its corresponding engagement feature may be provided on peripheral sidewall portions of the probe cover array and the support tray, respectively.

Alternatively or additionally, where the probe cover array comprises one or more support pins arranged between adjacent probe covers, one or more of the support pins may comprise an engagement feature configured to retain the probe cover array on the support tray. Where the support tray comprises one or more location points arranged between adjacent wells, for location of a support pin of the probe cover array, the location point may comprise a corresponding engagement feature. For example, one or more of (e.g. all of) the support pins may comprise a bulbous distal end, an 'arrowhead' shaped end, or a deformable hook or `snap-fit' member configured to engage with a lip or flange of the location point. Alternatively, one or more of (e.g. all of) the support pins may be configured to provide an interference fit between the pin (circular/square/triangular or other cross-section) and an elongate receptacle or aperture/hole/socket of the location point, e.g. a lateral dimension of the support pin may be selected to provide such an interference fit.

In some arrangements, a single probe cover array/support tray combination may comprise two or more different types of engagement features. For example, a single probe cover array/support tray combination may comprise both a cantilever snap-fit arrangement provided on peripheral sidewall portions of the probe cover array and the support tray respectively, as well as engagement features provided on support pins/location points of the probe cover array and support tray respectively.

The invention includes the combination of the aspects and preferred features described except where such a combination is clearly impermissible or expressly avoided. In particular, it is clear to the person skilled in the art that optional features set out above in relation to the probe cover array of the first aspect are equally applicable to the probe cover array of the second aspect, and vice versa.

### Summary of the Figures

Embodiments and experiments illustrating the principles of the invention will now be discussed with reference to the accompanying figures in which:
Figure 1 is an isometric line drawing of a probe cover array according to the present invention.
Figure 2 is an isometric line drawing of a support tray for a probe cover array according to the present invention.
Figure 3 is an isometric line drawing of the probe cover array and support tray of Fig. 1 and Fig. 2 respectively provided as a joined assembly.
Figure 4 is a detail view of a portion of the joined assembly including a probe cover array and support tray shown in Fig. 3.
Figure 5 is a plan view of the joined assembly including a probe cover array and a support tray shown in Fig. 3.
Figure 6 is a cross-sectional line drawing of the joined assembly including a probe cover array and a support tray, taken along line A-A shown in Fig. 5.
Figure 7 is a cross-sectional line drawing of the joined assembly including a probe cover array and a support tray, taken along line B-B shown in Fig. 5.
Figure 8 is an isometric line drawing of a further embodiment of a probe cover array and support tray according to the present invention, shown in an exploded arrangement.
Figure 9 is a partial perspective cross-sectional view of the probe cover array and support tray of Fig. 8 shown in an exploded arrangement.
Figure 10 is a partial cross-sectional view of the probe cover array and support tray of Fig. 8, showing engagement of the support pins of the probe cover array with the location points of the support tray.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

A brief description of the figures is provided above. The figures show various illustrations of a first embodiment of a probe cover array 100, a first embodiment of a support tray 200, and the interaction between said probe cover array and support tray.

The probe cover array 100 is an integrally-moulded polymeric component comprising a frame 1, and a plurality of probe covers 3. In the embodiment shown, the probe covers are arranged in three staggered rows. The external rows (the first and third rows) comprise seven probe covers. The internal row (the second row) comprises six probe covers. In the embodiment shown, the rows are staggered by arranging the probe covers such that the longitudinal axes of probe covers (not visually indicated) in the first and third rows are aligned with the midpoint between two directly adjacent probe covers in the second, directly adjacent, central row. Here, the longitudinal axes of probe covers are arranged approximately vertically (i.e. perpendicular to the plane in which the array of probe covers extends).

The probe covers are configured for engagement with a tympanic probe (not shown). Each probe cover 3 is generally frusto-conical in shape, comprising a tubular body portion 5, the body having a proximal end 7 defining an opening configured for receipt of a distal end of a thermometer, and a distal end 9, distal to the proximal end. The diameter of the tubular body portion at the proximal end 7 is about 12 mm (internal diameter) or 14 mm (external diameter). The diameter of the tubular body portion at the distal end 9 is about 5.6 mm (internal diameter) or 7 mm (external diameter). It has been found that this is a convenient shape for probe covers, as the frusto-conical shape can allow the probe cover to be partially inserted into ear canals of different sized patients - for example, both children and adults. Furthermore, the frusto-conical shape may also allow the probe cover to closely fit a thermometer probe such as a tympanic thermometer probe, which can assist in retention of the probe cover on the thermometer probe during use.

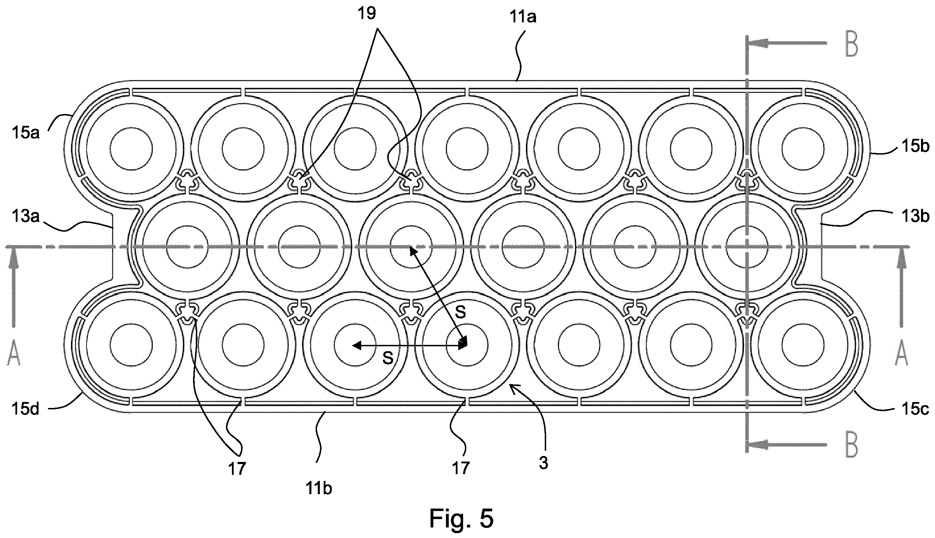

The frame 1 of the probe cover array comprises peripheral sidewalls including two longitudinal wall portions 11a, b, which extend in a direction substantially parallel to the rows of probe covers, and two lateral wall portions 13a, b, which extend in a direction substantially perpendicular to the rows of probe covers. The peripheral sidewalls further comprise curved wall portions 15 a, b, c, d (indicated in Fig. 5) connecting the longitudinal wall portions and the lateral wall portions.

The probe covers 3 are connected to the frame 1 of the probe cover array via a plurality of frangible links 17. Here, the frangible links are conveniently provided as a web of material integrally formed with the rest of the probe cover array. It can be seen that some frangible links directly connect some probe covers to the frame of the array (e.g. those probe covers forming part of external first and third rows of probe covers each have at least one frangible link directly connecting the probe cover to the frame). However, other frangible links are provided which indirectly connect given probe covers to the frame. For example, probe covers forming part of the internal second row are indirectly connected to the frame via first and second frangible links, each of which attaches indirectly to two further probe covers in the external first or third rows via a support pin 19, said further probe covers being directly attached to the frame by further frangible links.

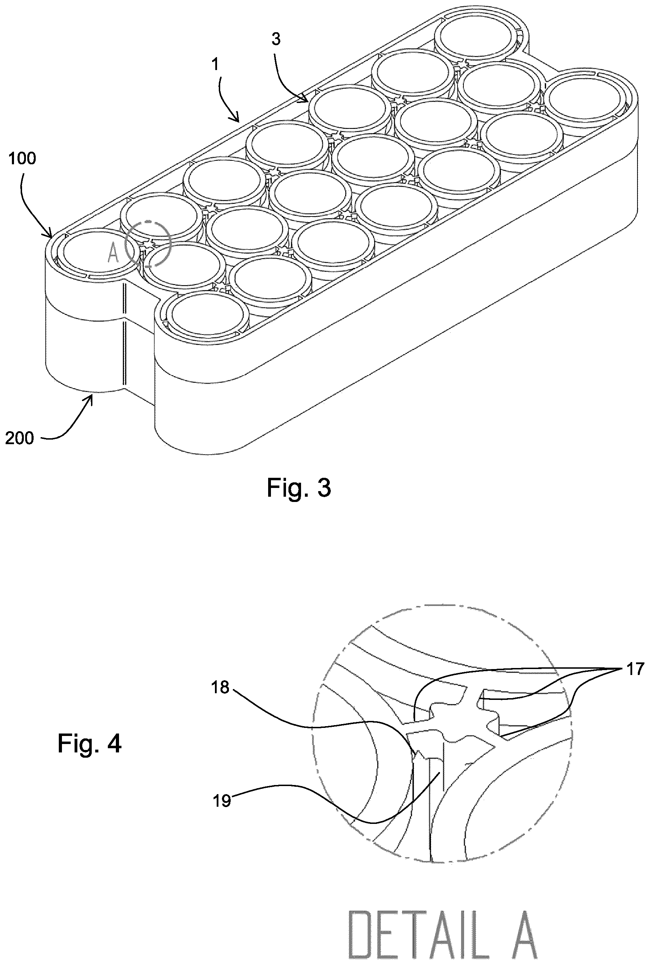

The frangible links 17 are relatively small in relation to the size of the probe covers and are configured to break on application of a predetermined force which can be referred to as a 'detachment force'. In this way, as the probe covers are attached to the frame of the probe cover array via frangible links, the connection of the probe covers to the frame via the links can support the probe covers during storage, but the probe covers can readily be detached by a user by application of a detachment force (for example, when inserting a thermometer probe into a given probe cover for use). As can be seen in Fig. 4, the frangible links can comprise a mechanical stress raiser feature - in the embodiment of Fig. 4, a notch 18 is provided on an underside of the frangible links. This presence of this notch may reduce the force needed to break the frangible links.

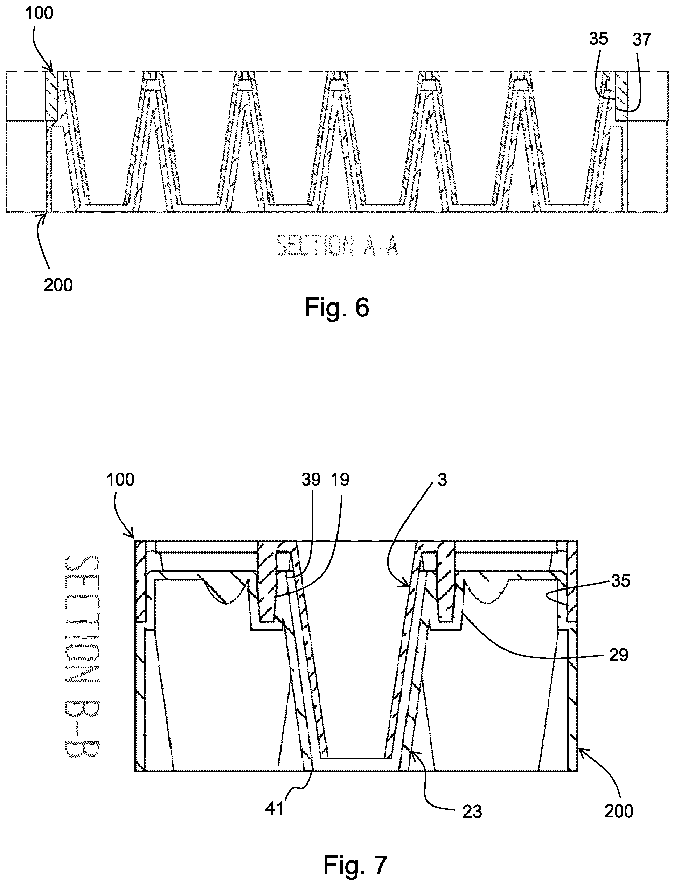

As mentioned above, the probe cover array further comprises a plurality of support pins 19, best seen in Fig. 4 and Fig. 7. In this arrangement, each support pin is provided at the junction between three adjacent probe covers. The support pins 19 comprise elongate members having a longitudinal axis which generally aligns with the longitudinal axis of a probe cover of the array of probe covers (i.e. which is arranged in a substantially vertical direction in the embodiment shown). The support pins are arranged to provide a reaction force when a detachment force is applied to a probe cover of the plurality of probe covers by engagement of the support pin with a supporting surface beneath the support pin. In the present embodiment, as shown in Fig. 7, the length of the support pins is less than the length of the probe covers (conveniently, they have a length of around 8 mm), and the supporting surface with which the support pins engage is provided the support tray 200, discussed in further detail below.

As shown in Fig. 4, the support pins have a generally triangular cross-sectional shape. This shape is preferred as it may provide a suitably high second moment of area of the support pin in a lateral axis, thereby providing for increased bending stiffness of the support pins. In the present embodiment, the support pins are quasi-prismatic in shape, in that they have a geometrically similar cross-sectional shape along the length of the pins, however the cross-sectional area of the support pins changes along the length of the pins, with the pins being slightly tapered towards a distal end of the pins - this can be seen in Fig. 7.

Regarding the arrangement of the probe covers 3 in the probe cover arrays, Fig. 5, which is a plan view of a probe cover array and a support tray, gives the best overview of this. In this figure, the centre-to centre spacing between a number of adjacent probe covers is indicated with a double-ended arrow and the reference numeral 'S'. The centre of each probe cover is taken as a point on the longitudinal central axis of the probe covers. Conveniently, the centre-to-centre distance between probe covers is measured in a plane parallel to the surface of the probe cover array. It can be seen from Fig. 5 that the center-to-center spacing between each given probe cover and all probe covers directly adjacent said given probe cover is substantially equal, and that the probe covers can be said to be arranged in a hexagonal-close-packed arrangement (or, at least pseudo hexagonally-close-packed). In this arrangement, the center-to-center spacing between each given probe cover and all probe covers directly adjacent said given probe cover is about 1.25 times the diameter of the given probe cover, as measured at the widest point of the probe cover (which lies in the plane parallel to the surface of the probe cover array). This minimum distance is required to accommodate the presence of frangible links 17 and support pins 19 which are located in the void space between adjacent probe covers. However, it has been found that this specific arrangement allows for good packing efficiency of the probe cover array, whilst ensuring there is no requirement for laterally- or longitudinally-extending bracing elements (e.g. cross-bars or walls) to be located between the probe covers in the array, thereby reducing the amount of material required for manufacture of the probe cover array.

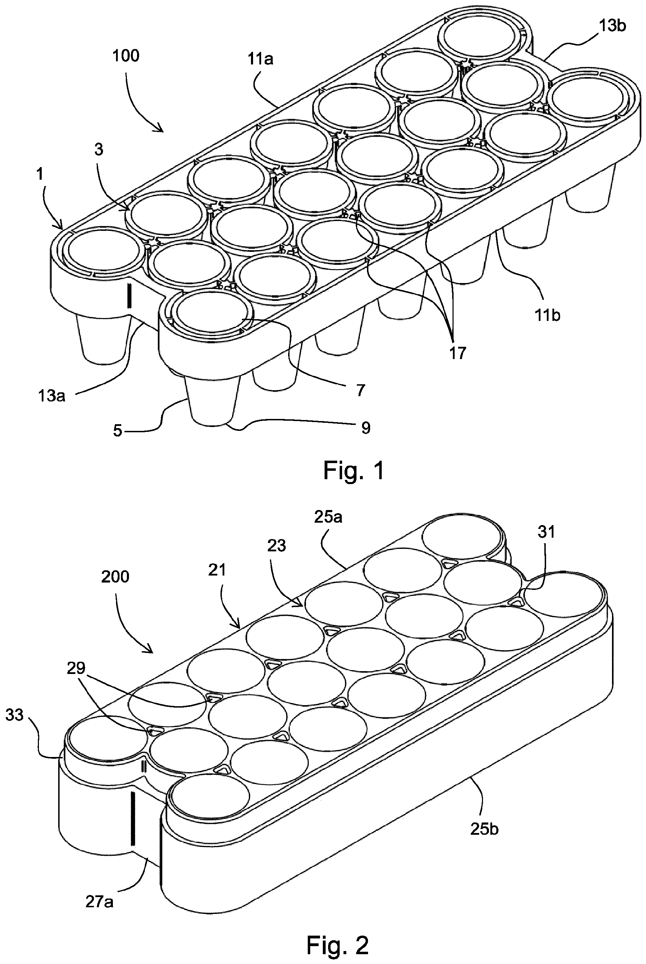

The configuration of the support tray 200 will now be discussed. This is best discussed at least initially in relation to Fig. 2, which is an isometric line drawing of the support tray. The support tray 200 comprises a support tray body 21 and a plurality of wells 23 formed in the support tray body. Each well is configured for receipt of a probe cover, and the support tray is configured to receive and support the probe cover array 100 shown in Fig. 1, as will be discussed in further detail below.

It can be seen from these figures that the number and arrangement of wells 23 in the support tray substantially corresponds to the number and arrangement of probe covers 3 in the probe cover array 100. That is, in the embodiment shown, the wells 23 are arranged in three staggered rows. The external rows (the first and third rows) comprise seven wells. The internal row (the second row) comprises six wells. The rows of wells are staggered by arranging the wells such that the longitudinal axes of the wells (not visually indicated) in the first and third rows are aligned with the midpoint between two directly adjacent wells in the second, directly adjacent, central row. Here, the longitudinal axes of the wells are arranged approximately vertically (i.e. perpendicular to the plane in which the array of wells extends). In a corresponding manner as for the probe cover array, the center-to-center spacing between each given well and all wells directly adjacent said given well is substantially equal, and is about 1.06 times the diameter of the given well.

The support tray 200 comprises peripheral sidewalls including two longitudinal wall portions 25a, b, which extend in a direction substantially parallel to the rows of probe covers, and two lateral wall portions 27a, b (27b not visible) which extend in a direction substantially perpendicular to the rows of probe covers. The peripheral sidewalls further comprise curved wall portions connecting the longitudinal wall portions and the lateral wall portions.

The support tray further comprises a plurality of location points which are conveniently provided as sockets 29 arranged between adjacent wells 23, each socket being configured for location of a corresponding support pin 19 of the probe cover array. In this arrangement, each socket is provided at the junction between three adjacent wells.

As can be seen in Fig. 7, the sockets 29 have a depth less than the length of the support pin(s). In this way, the support pins can engage the bottom of the socket when they are located in the socket, and the bottom of the socket therefore acts to provide a supporting surface beneath the support pin which can provide a reaction force when a detachment force is applied to a probe cover of the plurality of probe covers by engagement of the support pin with said supporting surface.

The shape of the sockets is selected to correspond to the shape of the support pins 19. In this arrangement, the sockets therefore also have a generally triangular shape. One advantage of the use of triangular pins/sockets, is that the pins can only be located in the sockets in a selected number of orientations. This can therefore assist with correctly aligning the probe cover array with the support tray.

Each socket further also comprise a sloped lead-in surface 31 surrounding the socket aperture. Provision of a sloped lead-in surface can assist in location of the support pin(s) in the location point(s) during alignment of the probe cover array and the support tray.

The support tray further comprises a recessed portion 33 which extends around the periphery of the support tray (best seen in Fig. 2). The recessed portion 33 is shaped such that a surface 35 of the recessed portion conforms with an inner sidewall surface 37 of the peripheral sidewalls of the probe cover array 100 - see Fig 6 and Fig. 7. In this way, the support tray is configured to provide a close-fit arrangement (e.g. an interference fit) between the support tray and the probe cover array. This allows the probe cover array and the support tray to be readily assembled to provide a joined assembly having a relatively stable connection between the probe cover array and the support tray.

Fig. 6 and Fig. 7 also show the shape of the wells 23 of the support tray in greater detail. It can be seen that the wells have a generally similar shape to the probe covers of the probe cover array. That is, each well 23 is generally frusto-conical in shape, and has a proximal end 39 defining an opening configured for receipt of a probe cover 3, and a distal end 41, distal to the proximal end. In this arrangement, the distal end is open. The (internal) diameter of each well at the proximal end 39 is about 14.1 mm. The (internal) diameter of each well at the distal end 41 is about 8.5 mm.

In the present embodiment, the walls of the support tray (including walls formed the wells) are shown as being solid. However, it is contemplated that in some arrangements, it may be desirable to remove some material from the walls of the support tray (e.g. by provision of one or more apertures in the walls forming the support tray), as this can allow for a reduced amount of material to be used in the manufacture of the support tray.

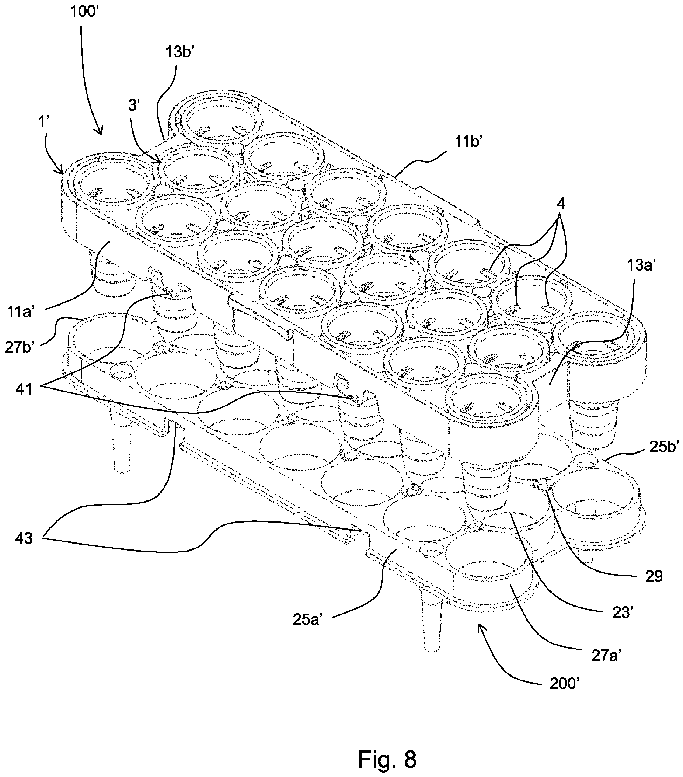

Figs. 8-10 depict further embodiments of a probe cover array and support tray (and kit comprising both of these) according to the present invention. The probe cover array and support tray are generally similar in form to the embodiments discussed in relation to Fig. 1-7, and so many features of these embodiments will not be discussed in detail - where specific details of this embodiment are not discussed below, refer to the above discussion of Fig. 1-7. Similarly to the above embodiments, the probe cover array 100' is an integrally-moulded polymeric component comprising a frame 1', and a plurality of probe covers 3'. The probe covers are arranged in three staggered rows, and the probe covers can be said to be arranged in a hexagonal-close-packed arrangement (or, at least pseudo hexagonally-close-packed). The probe covers are configured for engagement with a tympanic probe (not shown), with each probe cover 3' being generally frusto-conical in shape. In a departure from the arrangement shown in Fig. 1-7, each probe cover 3' comprises multiple engagement features for engagement with corresponding engagement features provided on the thermometer probe - these are provided as raised features 4 on probe cover body. The raised features are located at a proximal end of each probe cover body.

The frame 1' of the probe cover array comprises peripheral sidewalls including two longitudinal wall portions 11a', b', which extend in a direction substantially parallel to the rows of probe covers, and two lateral wall portions 13a', b', which extend in a direction substantially perpendicular to the rows of probe covers. The peripheral sidewalls further comprise curved wall portions connecting the longitudinal wall portions and the lateral wall portions. In this embodiment, the longitudinal wall portions 11 a', b' each comprise engagement features in the form of cantilever snap-fits 41. Each longitudinal wall portion comprises two such snap-fits. The cantilever snap-fits 41 are configured to engage with a corresponding engagement feature provided as recesses 43 formed in the sidewall of the support tray. The probe cover array and support tray can be 'snap-fitted' together using these cantilever snap-fits, thereby allowing the probe cover array to be retained on the support tray. This can both assist in efficient detachment of a single probe cover from the probe cover array during use by a user, as well as ensuring continued alignment of the probe cover array with the support tray during use.

The probe covers 3' are connected to the frame 1' of the probe cover array via a plurality of frangible links, although these are not clearly visualised in Fig. 8-10. Nevertheless, a similar arrangement applies as described above in relation to the embodiments shown in Fig. 1-7.

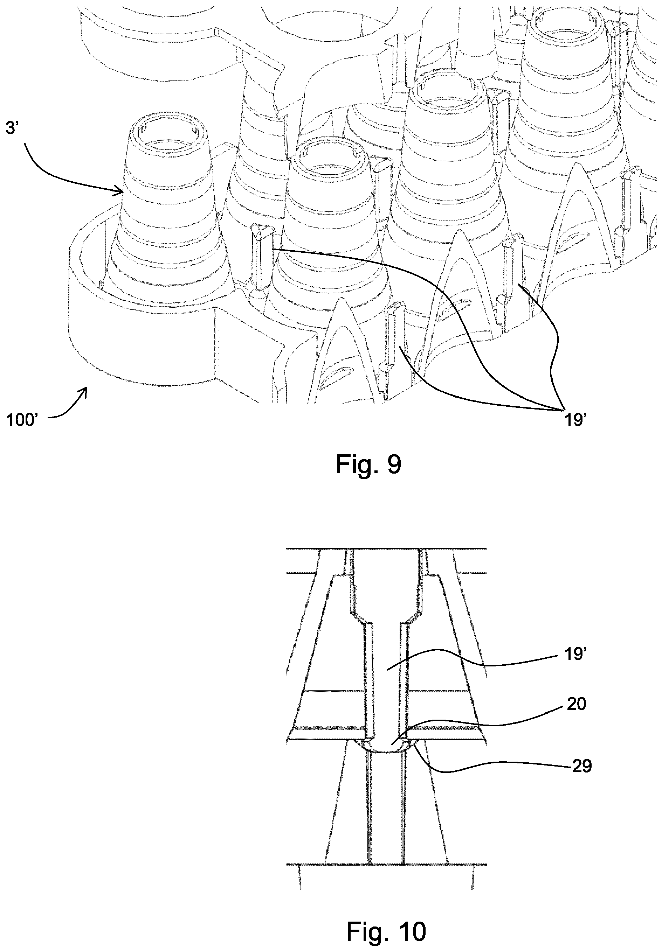

The probe cover array further comprises a plurality of support pins 19' (best seen in Fig. 9 and Fig. 10). Each support pin is provided at the junction between three adjacent probe covers. The support pins 19' comprise elongate members having a longitudinal axis which generally aligns with the longitudinal axis of a probe cover of the array of probe covers. As shown in Fig. 9, the support pins have a generally triangular cross-sectional shape, and are provided with a bulbous distal end portion 20 for engagement with the support tray in a manner discussed below.

The support tray 200' comprises a support tray body and a plurality of wells 23' formed in the support tray body. Here the wells are formed a through-holes passing through the support tray body. Each well is configured for receipt of a probe cover, and the support tray is configured to receive and support the probe cover array 100': the number and arrangement of wells 23' in the support tray substantially corresponds to the number and arrangement of probe covers 3' in the probe cover array 100'.

The support tray 200' comprises peripheral sidewalls including two longitudinal wall portions 25a', b', which extend in a direction substantially parallel to the rows of probe covers, and lateral wall portions 27a', b' which extend in a direction substantially perpendicular to the rows of probe covers. In this embodiment the lateral wall portions are curved to follow the shape of the wells of the support tray. As discussed above, the longitudinal wall portions 25a', b' each comprise recesses 43 to allow for snap-fit engagement of the probe cover array with the support tray.

The support tray further comprises a plurality of location points which are conveniently provided as sockets 29' arranged between adjacent wells 23', each socket being configured for location of a corresponding support pin 19' of the probe cover array. In this arrangement, each socket is provided at the junction between three adjacent wells.

The engagement between the support pins 19' of the probe cover array with the location points 29' of the support tray during use is shown in greater detail in Fig. 10. Here the bulbous distal end portion 20 of the support pin 19' can more clearly be seen. During use, this engages with a lip or flange of the socket 29, thereby retaining the support pin in the socket 29, thereby allowing the probe cover array to be retained on the support tray. This can both assist in efficient detachment of a single probe cover from the probe cover array during use by a user, as well as ensuring continued alignment of the probe cover array with the support tray during use. By providing engagement features both on the support pins as well as the frame of the probe cover array, a synergistic advantage is achieved, as the probe cover array is attached to the support tray at a plurality of locations during use, providing more effective retention of the probe cover array on the support tray. Whilst the engagement member on the support pin in shown as a bulbous portion in this embodiment, the skilled person will appreciate that other forms would provide similar effect: for example, the support pins may comprise an 'arrowhead' shaped end or may comprise a deformable hook or 'snap-fit' member e.g. at a location along the length of the pin.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

The following numbered paragraphs contain statements of broad combinations of the inventive technical features herein disclosed:
1. A probe cover array comprising a frame, and a plurality of probe covers connected to the frame via one or more frangible links, wherein the plurality of probe covers are arranged in at least two staggered rows, wherein the center-to-center spacing between each given probe cover and all probe covers directly adjacent said given probe cover is substantially equal.
2. The probe cover array according to paragraph 1 wherein the center-to-center spacing between each given probe cover and all probe covers directly adjacent said given probe cover is no greater than 1.3 times the diameter of the given probe cover.
3. The probe cover array according to paragraph 1 or paragraph 2 wherein the probe cover array comprises one or more support pins arranged between adjacent probe covers.
4. A probe cover array comprising a frame, and a plurality of probe covers connected to the frame via one or more frangible links, wherein the probe cover array comprises one or more support pins arranged between adjacent probe covers.
5. The probe cover array according to paragraph 4 wherein the plurality of probe covers are arranged in at least two staggered rows, wherein the center-to-center spacing between each given probe cover and all probe covers directly adjacent said given probe cover is substantially equal.
6. The probe cover array according to paragraph 5 wherein the center-to-center spacing between each given probe cover and all probe covers directly adjacent said given probe cover is no greater than 1.3 times the diameter of the given probe cover.
7. The probe cover array according to any one of paragraphs 3 to 6, wherein the support pins comprise elongate members, and the longitudinal axis of the support pin(s) generally aligns with the longitudinal axis of a probe cover of the array of probe covers.
8. The probe cover array according to any one of paragraphs 3 to 7 wherein the support pins comprise a generally triangular cross-sectional shape.
9. A probe cover array according to any one of paragraphs 3 to 8 wherein the length of the support pins is less than the length of the probe covers.
10. The probe cover array according to any one of the preceding paragraphs, wherein the array comprises at least three rows of probe covers.
11. The probe cover array according to any one of the preceding paragraphs wherein each probe cover is attached to the frame via at least two frangible links
12. The probe cover array according to any one of the preceding paragraphs wherein the frangible links comprise a mechanical stress raiser feature.
13. The probe cover array according to any one of the preceding paragraphs wherein each probe cover comprises a tubular body portion, the body having a proximal end defining an opening configured for receipt of a distal end of a thermometer, and a distal end, distal to the proximal end.
14. The probe cover array according to paragraph 13 wherein the tubular body portion is tapered from the proximal end to the distal end.
15. The probe cover array according to any one of the preceding paragraphs where the probe cover array is an integrally molded component.
16. A support tray comprising a body and a plurality of wells formed in the body, each well being configured for receipt of a probe cover, wherein the support tray is configured to receive and support a probe cover array according to any one of the preceding paragraphs.
17. The support tray according to paragraph 16 wherein the center-to-center spacing between each given well and all wells directly adjacent said given well is substantially equal.
18. The support tray according to paragraph 17 wherein the center-to-center spacing between each given well and all wells directly adjacent said given well is no greater than 1.3 times the diameter of the given well.
19. The support tray according to any one of paragraphs 16 to 18 wherein the tray comprises one or more location points arranged between adjacent wells.
20. The support tray according to paragraph 19 wherein one or more of the location points comprise a depression, socket, or through-hole formed in the support tray body.
21. The support tray according to paragraph 19 or 20 wherein the support tray comprises sloped lead-in surfaces surrounding each location point.
22. The support tray according to paragraph 20 or 21, wherein each of the location points comprises a socket having a generally triangular cross-sectional shape.
23. The support tray according to any one of paragraphs 16 to 22 wherein the support tray comprises a recessed portion which extends around the periphery of the support tray.
24. The support tray according to any one of paragraphs 16 to 23 wherein the support tray is an integrally molded component.
25. A kit of parts comprising a support tray according to any one of paragraphs 16 to 24, and one or more probe cover arrays according to any one of paragraphs 1 to 15.
26. The kit according to paragraph 25 wherein the probe cover array and/or the support tray comprise one or more engagement feature(s) configured to retain the probe cover array on the support tray during use.
27. The kit according to paragraph 26 wherein one of the probe cover array and the support tray comprises a cantilever snap fit configured to engage with a corresponding engagement feature provided on the other of the probe cover array and support tray, optionally wherein the cantilever snap fit and the corresponding engagement feature are provided on peripheral sidewall portions of the probe cover array and the support tray, respectively.
28. The kit according to paragraph 26 or paragraph 27 wherein the probe cover array comprises one or more support pins arranged between adjacent probe covers, the support tray comprises one or more location points for location of the support pins arranged between adjacent wells, and wherein one or more of the support pins comprises an engagement feature configured to retain the probe cover array on the support tray by engagement with a corresponding engagement feature provided by a corresponding location point.
29. The kit according to paragraph 28 wherein the engagement feature of the support pin is selected from the following:
   (i) a bulbous distal end;
   (ii) an 'arrowhead' shaped end;
   (iii) a deformable hook or `snap-fit' member; or
   (iv) a lateral dimension selected to provide an interference fit with at least part of the corresponding location point.

## Claims

1. A probe cover array comprising a frame, and a plurality of probe covers connected to the frame via one or more frangible links, wherein the probe cover array comprises one or more support pins arranged between adjacent probe covers.

2. The probe cover array according to claim 1 wherein the plurality of probe covers are arranged in at least two staggered rows, and wherein the center-to-center spacing between each given probe cover and all probe covers directly adjacent said given probe cover is substantially equal, optionally wherein the center-to-center spacing between each given probe cover and all probe covers directly adjacent said given probe cover is no greater than 1.3 times the diameter of the given probe cover.

3. The probe cover array according to claim 1 or claim 2, wherein the support pins comprise elongate members, and the longitudinal axis of the support pin(s) generally aligns with the longitudinal axis of a probe cover of the array of probe covers.

4. The probe cover array according to any one of claims 1 to 3 wherein the support pins comprise a generally triangular cross-sectional shape.

5. The probe cover array according to any one of the preceding claims wherein the frangible links comprise a mechanical stress raiser feature.

6. The probe cover array according to any one of the preceding claims wherein each probe cover comprises a tubular body portion, the body having a proximal end defining an opening configured for receipt of a distal end of a thermometer, and a distal end, distal to the proximal end.

7. The probe cover array according to claim 6 wherein the tubular body portion is tapered from the proximal end to the distal end.

8. The probe cover array according to any one of the preceding claims where the probe cover array is an integrally molded component.

9. A kit of parts comprising one or more probe cover arrays according to any one of claims 1 to 8, and support tray configured to receive and support the probe cover array(s), the support tray comprising a body and a plurality of wells formed in the body, each well being configured for receipt of a probe cover.

10. The kit of parts according to claim 9 wherein the support tray comprises one or more location points arranged between adjacent wells, optionally wherein one or more of the location points comprise a depression, socket, or through-hole formed in the support tray body.

11. The kit of parts according to claim 10 wherein the support tray comprises sloped lead-in surfaces surrounding each location point.

12. The kit according to any one of claims 9 to 11 wherein the probe cover array and/or the support tray comprise one or more engagement feature(s) configured to retain the probe cover array on the support tray during use.

13. The kit according to claim 12 wherein one of the probe cover array and the support tray comprises a cantilever snap fit configured to engage with a corresponding engagement feature provided on the other of the probe cover array and support tray, optionally wherein the cantilever snap fit and the corresponding engagement feature are provided on peripheral sidewall portions of the probe cover array and the support tray, respectively.

14. The kit according to claim 12 or claim 13 wherein the probe cover array comprises one or more support pins arranged between adjacent probe covers, the support tray comprises one or more location points for location of the support pins arranged between adjacent wells, and wherein one or more of the support pins comprises an engagement feature configured to retain the probe cover array on the support tray by engagement with a corresponding engagement feature provided by a corresponding location point.

15. The kit according to claim 14 wherein the engagement feature of the support pin is selected from the following:
(i) a bulbous distal end;
(ii) an 'arrowhead' shaped end;
(iii) a deformable hook or `snap-fit' member; or
(iv) a lateral dimension selected to provide an interference fit with at least part of the corresponding location point.
